Europäisches Patentamt

(19) European Patent Office

Office européen des brevets

(11) Publication number: **0 175 966**
**B1**

## (12) EUROPEAN PATENT SPECIFICATION

(45) Date of publication of patent specification: **24.01.90**

(21) Application number: **85111126.0**

(22) Date of filing: **04.09.85**

(51) Int. Cl.⁵: **C 12 N 15/00,** A 01 H 1/00,
C 12 N 1/08

(54) **Plant cell microinjection technique.**

(30) Priority: **25.09.84 US 654606**

(43) Date of publication of application:
**02.04.86 Bulletin 86/14**

(45) Publication of the grant of the patent:
**24.01.90 Bulletin 90/04**

(84) Designated Contracting States:
**AT BE CH DE FR GB IT LI LU NL SE**

(56) References cited:
**WO-A-83/01176**

**PROTOPLASMA, vol. 116, 1983 (Wien, 1 New York); H.-H.STEINBISS et al.: "Protoplast Derived Tobacco Cells Can Survive Capillary Microinjection of the Fluorescent Dye Lucifer Yellow", pp. 223-227**

**SCIENCE, vol. 151, no. 3708, 21 Jan 1966, WASHINGTON, DC (US); T.P.LIN: "Microinjection of mouse eggs", pp. 333-337**

(73) Proprietor: **CALGENE, INC.**
**1920 Fifth Street Suite F.**
**Davis California 95616 (US)**

(72) Inventor: **Crossway, Anne**
**1902 El Paso Avenue, No. 4**
**Davis California 95616 (US)**
Inventor: **Facciotti, Daniel**
**2636 Lafayette**
**Davis California 95616 (US)**

(74) Representative: **Glawe, Delfs, Moll & Partner**
**Patentanwälte**
**Postfach 26 01 62 Liebherrstrasse 20**
**D-8000 München 26 (DE)**

Courier Press, Leamington Spa, England.

**Description**

1. Field of the Invention

The ability to transfer exogenous genetic material into higher plants promises to provide great opportunities for agricultural scientists to increase food production in the coming decades. At present, the primary focus of those interested in the genetic manipulation of higher plants has been in two areas: use of the Ti plasmid of *Agrobacterium tumefaciens* and use of vectors based on the Caulimoviruses. While these systems offer the potential to integrate exogenous DNA into the plant's genome, they each suffer from particular drawbacks including limited plant host range and limited efficiency of transformation. It would thus be desirable to have an improved system for the genetic manipulation of plants which is free from these drawbacks.

Direct microinjection of DNA as practiced in animal cells has many advantages, including simplicity and very high transformation rates. Despite these advantages, the utilization of direct microinjection of DNA into plant cells has found only limited use. Direct microinjection of plant cells is complicated by the presence of a rigid cell wall not found in animal cells. While protoplasts lacking the cell wall can be formed, the microinjection of plant cell protoplasts is made difficult by their extreme fragility. Successful micro-injection has been achieved by immobilizing plant protoplasts on a solid substrate. Such immobilization, however, prevents easy separation of injected and non-injected cells, and the small percentage of injected cells can only be followed for a few days. A strong selectable marker would be required to allow identification and recovery of transformed cells, and the use of such markers may result in the loss of a significant portion of the transformed material.

Thus, it would be desirable to provide a method for the direct microinjection of DNA and other macromolecules into plant protoplasts, which method provides a high rate of uptake of the injected DNA into specified compartments of the plant cell with high viability of the injected protoplasts.

2. Description of the Prior Art

Steinbiss and Stabel (1983) Protoplasma 116:223—227, teach the microinjection of macromolecules into plant protoplasts where the protoplasts are first attached to microscope cover slips using polysine. Although functional, the attachment with polysine reduces the protoplasts viability and microinjection into the nucleus occurs infrequently. Griesbach (1983) P1. Mol. Biol. Rep. 4:32—37 discloses the microinjection of chromosomes into higher plant protoplasts that are either free-floating or suspended in agar. Injection into the nucleus was not obtainable and protoplast viability was severely reduced. The microinjection of DNA into mouse eggs using a holding pipette and a microinjection pipette is described in Lin (1966) Science 151:333—337. See also, Wagner et al. (1981) Proc. Natl. Acad. Sci. USA 78:6376—6380; Brinster et al. (1981) Cell 27:223—231; and Gordon and Ruddle (1981) Science 214:1244—1246, where the successful transfer and expression of genetic material into the genomes of newborn mice by microinjection is described.

Summary of the Invention

An improved method for microinjecting DNA and other macromolecules into plant cell protoplasts is provided. The method is characterized by preculturing the protoplasts for a period of time sufficient to partially regenerate the cell wall. Such partial regeneration of the cell wall strengthens the protoplast during subsequent manipulations and greatly enhances the viability of injected protoplasts. Injection of the DNA or other macromolecule is accomplished using a specially formed injection pipette while the cells are immobilized. In the preferred embodiment, individual protoplasts are held by suction using a holding pipette while the injection pipette is inserted into the protoplast. Injected protoplasts are collected together and cultured in isolation from other protoplasts and cells. In this way, the progress of injected protoplasts can be easily followed.

Description of the Specific Embodiments

The present invention provides a novel method for injecting macromolecules, typically polynucleotides, isolated chromosomes, and the like, as well as organelles, such as nuclei, into plant protoplasts. The method is characterized by culturing freshly prepared protoplasts for an amount of time sufficient to partially regenerate the cell wall prior to injection of the macromolecules. The partial cell wall provides sufficient mechanical support to preserve the protoplast during the injection, while allowing penetration by the injection means, typically an injection pipette.

In the preferred embodiment, the partially-regenerated protoplasts are individually manipulated using a holding pipette which is adapted to hold the protoplast by applying a small suction on the partially regenerated cell wall. Using the holding pipette, the user is able to orient the protoplast to expose the nucleus. An injection pipette is then used to penetrate the protoplast and inject the macromolecule directly into the nucleus. The ability to inject directly into the nucleus is important to allow transformation of protoplasts by injection of DNA. The injected protoplasts are then cultured separately to allow the injected cells and resulting culture to be observed in isolation from non-injected cells.

The method of the present invention is useful with protoplasts formed from virtually any higher plant. In particular, the method is useful with higher plant protoplasts which possess totipotency and from which the entire plant can be regenerated. Current methods exist for protoplast regeneration from various species of *Solanaceae*, such as *Solanum tuberosum* (potato), *Lycopersicum esculentum* (tomato), *Nicotiana tabacum* (tobacco), *Solanum melogena* (eggplant), *Capsicum annuum* (peppers), and various petunias, e.g., *Petùnia hybrida*. In addition to these, other valuable economic crops, such as cereals, wood plants, legumes, and the like, have been regenerated from protoplasts under particular conditions. It is expected that the ability to regenerate whole plants from plant protoplasts will improve as the particular conditions for such regeneration, e.g., plant hormones, protoplast density, pH, light, and the like, are identified.

The method of the present invention is particularly useful for the modification of higher plants by transformation of the protoplast and regeneration of the protoplast into the whole plant. A wide variety of genes and other DNA including isolated chromosomes and nuclei may be introduced into the protoplast to become integrated into the plant genome. The DNA may be "bare" or incorporated into a vector system, e.g., systems based on the Ti plasmid or the Caulimoviruses. The genes introduced may provide for a wide variety of modifications. By introducing genes which control various functions, the functions of the plant can be widely varied. Plant growth can be inhibited or enhanced. Nutrient requirements may be modified. Production of various plant products can be increased or decreased. Enhanced protein and/or saccharide content can be provided. The plant can be adapted to survive in hostile environments, such as reduced light, lower temperature, brackish water. Protection against bacterial and pest infection can also be provided. These and other modifications can be achieved by providing genes which produce the particular proteins responsible for these characteristics.

Protoplasts may be formed by enzymatically digesting the cell wall of a desired plant cell using a cellulase, typically a fungal cellulase. For example, plant leaves may be macerated, the main veins discarded and the macerate treated with a solution of an osmoticum and the cellulase. The resulting protoplasts may be pelleted and washed to remove the cellular debris. A specific method for forming tobacco mesophylls is set forth in the Experimental section hereinafter. Other methods for forming plant protoplasts are well known in the art.

The freshly prepared protoplasts are cultured in a suitable nutrient medium for an amount of time sufficient to partially regenerate the cell wall to facilitate subsequent manipulation. It has been found that the partially regenerated cell wall does not inhibit penetration by the injection pipette into the protoplasts. The amount of time required for regeneration will vary depending on the cell type, the culturing conditions, and the like. Injection should be performed prior to complete regeneration of the cell wall (which occurs immediately prior to first cell division) since the regenerated cell wall blocks penetration of the injection pipette. Typically, the regeneration time will be at least one day and will not exceed five days. More typically regeneration time will be between one and three days. It may be desirable, although not necessary, to determine the extent of cell wall regeneration by the method of Nagata and Takebe, Planta (Berl) (1970) 92:301—308. Using this method, the time required for cell wall regeneration under the particular conditions may be determined. Microinjection may then be timed to occur when the cell walls have substantially regenerated in a majority of the protoplasts, but before first cell division.

Prior to protoplast injection, it is necessary to immobilize the cell so that the injection pipette can be inserted through the partially-regenerated cell wall. A holding pipette (as described below) is utilized to grasp an individual protoplast from the culture and to manipulate said protoplast during injection. Injection is accomplished under aseptic conditions using an ultrafine injection pipette. Conveniently, to maintain aseptic conditions, a drop of the protoplast suspension and a drop of the macromolecule solution, e.g., DNA solution, are formed adjacent one another and covered with oil. In the exemplary embodiment, this is accomplished using a microscope slide having a depression to contain the oil. The holding pipette and injection pipette are then manipulated using commercially available manipulators while viewing under the microscope. The protoplasts will be oriented to expose the nucleus to allow direct injection of the DNA into the nucleus.

The holding pipette may be prepared from commercially available capillary tubing, typically 1.0 millimeter tubing, using a pipette puller in the conventional manner. The holding pipette is first pulled to form a tapered end, and the taper is broken at a particular orifice diameter depending on the size of the protoplasts. The tip is broken by annealing, typically using a heated glass bead, followed by rapid cooling. The broken surface is then fire-polished by holding it close to the heated filament. The orifice diameter of the pipette should be equal to about one quarter the diameter of the cell. Thus, for a typical tobacco cell protoplast having a diameter of about 40 microns, the orifice of the holding pipette should be about 10 microns. The remote end of the pipette may be bent to facilitate observation during manipulations within the depression slide.

The injection pipette is also formed by pulling a standard glass capillary tube on a commercially available pipette puller. The tube is pulled until it breaks at its narrowest point, leaving an extremely fine tapered tip. The tip of the pipette is on the order of one micron in diameter. The injection pipette may be calibrated by measuring the length and basal diameter of the portion of the pipette tip which will hold the injection solution. Estimation of the volume injected is then made by observing the shift in the meniscus between the DNA solution and the oil and applying the appropriate geometric formula. To facilitate introduction of larger structures, such as chromosomes, nuclei, and other organelles, the tip of the injection pipette may be internally beveled to increase the internal orifice diameter without increasing the outside diameter.

The holding and injection pipettes are filled with a viscous oil, typically paraffin oil, and inserted into suitable micromanipulators. The remote ends of the pipettes are connected to syringes by plastic tubing also filled with oil. Thus, the pressure within the pipette can be manipulated by adjusting the syringes. The depression slide holding the DNA and protoplast solution is placed underneath the viewing microscope and both the holding pipette and injection pipette are brought into focus. Before immersing the tips of the pipettes beneath the oil layer, the tip of the injection pipette is removed by touching it against the holding pipette, leaving an opening of about one micron.

The injection pipette is next inserted into the DNA drop, and approximately 10 pL of the DNA is drawn in using the associated syringe. The injection pipette is then removed from the DNA drop, and both the injection pipette, and the holding pipette are lowered into the protoplast solution. Protoplasts are grasped individually by holding the orifice of the holding pipette adjacent the protoplast and applying a small suction using the associated syringe. After raising the protoplast, the orientation of the protoplast may be adjusted by gentle turning using the injection pipette. Once the nucleus is exposed, the injection pipette may be inserted into the nucleus, and a desired volume of DNA solution, typically 1 to 2pL, injected.

Following injection of the DNA, the protoplasts are regenerated into callus tissue by culturing in an aseptic environment on a phytohormone-containing culture medium. Calli are forced to regenerate into plantlets by stimulation with a hormone, e.g., cytokinin, for a short period of time, e.g., one to three days. The genetically-modified plantlets may then be potted in a sterile potting mix and permitted to grow.

The presence of the desired gene in the plant cells can then be established in a wide variety of ways, depending on the nature of the gene. The presence of a gene which produces an exogenous product may be detected by isolation and lysis of the plant cell and an analysis of the cytoplasm for the exogenous product, or of the nucleus for the exogenous gene. The exogenous product may be detected by electrophoresis, chromatography, immunoassay, or the like. The gene can be detected conveniently by hybridization, for example, by using Southern Blotting.

Once a plantlet or plant has been shown to have been transformed, the cells of the plant may then be used repeatedly for tissue culture, followed by the growth of plantlets. Thus, the modified plant can be repetitively regenerated by use of cell and tissue culture. In some instances, propagation may be maintained from seed, although monitoring for loss of the exogenous gene would be advisable.

To facilitate identification of transformed protoplasts, it is desirable that the injected protoplasts be cultured in isolation from non-injected protoplasts. To do so, however, requires microculture techniques since plant protoplasts typically require a minimum density of $10^4$ to $10^6$ cells per milliliter. By employing very small culture volumes, the density of injected cells can be kept within the requisite range without requiring a very large number of transformed protoplasts.

A particular technique for the microculture of protoplasts is accomplished by forming a hanging drop of culture solution on a plate inverted over a suitable culture solution. Conveniently, a petri dish lid can be used and inverted over a petri dish including the culture media. The enclosed system is maintained at the proper temperature, and the resulting humidity inhibits the evaporation of media from the hanging drops. The size of the hanging drop is dependent upon the number of protoplasts, with the drop having an estimated volume in the range from about 0.25 to 0.5 microliters typically carrying from about 20 to 40 protoplasts.

Growth of the hanging drop cultures may be monitored under a microscope, and medium added as the microcalli grow. Media is replenished or changed using a small pipette. When the calli reach a sufficient size, typically about 1.0 mm in diameter, they may be transferred to agar plates containing a suitable medium. Plantlets may be regenerated from the microcalli using conventional techniques, as described above.

The following examples are offered by way of illustration, not by way of limitation. The following abbreviations are used:

BAP — Benzylaminopurine
IAA — Indole acetic acid
NAA — Naphthalene acetic acid

## Experimental

*Materials and Methods*

1. Preparation of Tobacco Mesophyll Protoplasts

Protoplast donor plants of *Nicotiana tabacum* cv. Xanthia were grown in glass jars under aseptic conditions as described by Facciotti and Pilet (1979) Pl. Sci. Lett. 15:1—6. Apical shoots were placed into 100 ml of agar medium (0.6% Gibco Phytagar MS medium containing 30.0 g/l sucrose, 1.0 mg/l IAA and 0.15 mg/l kinetin, pH adjusted to 5.75 prior to autoclaving). The cultures were kept at 23±2°C under a 12 hour dark/light regime. Young leaves were removed from 2—3 week old plants, the main veins discarded, and the leaf blades infiltrated in a 6% sorbitol solution with 0.04% pectinase (Pectolylase Y—23) and 0.4% cellulase (Onozuka RS). After 2—3 hours incubation, the macerate was passed through a 149μ nylon filter. The protoplasts were pelleted by centrifugation at 50 g and washed twice with 6% sorbitol solution. The protoplasts were then suspended at a density of 1—2 × $10^5$/ml in modified MS medium (0.5 MS concentration, Gibco 510—1118, plus 5.0 g/l sucrose, 71.0 g/l sorbitol) with 3.0 mg/l NAA and 1.0 mg/l BAP as described by Caboche (1980) Planta 194:7—18.

4

Preculturing prior to injection was performed in 9.0 cm Parafilm-sealed petri dishes at 23±2°C in the dark for 2—5 days.

### 2. Slide Preparation

All procedures were performed in a laminar flow hood. Depression slides were made by gluing a coverslip over a square hole cut into a plexiglass slide. When inverted, a depression was formed in which all manipulations were performed.

Protoplasts were selected under a dessecting microscope from the cultures prepared, as described above. Selection and transfer of protoplasts were performed with a glass transfer pipette connected by plastic tubing to a micrometer syringe. Transfer pipettes were pulled by hand over a bunsen burner from 1.2 mm O.D. glass tubing, resulting in an orifice of about 100 to 150 μ.

A drop of medium containing the selected protoplasts was formed in the depression on the slide. A second drop comprising the DNA solution to be injected was also formed in the depression. A variety of DNA constructs were injected into an aqueous solution. The depression was then filled with paraffin oil covering both the protoplast and DNA solutions to prevent evaporation and contamination during the subsequent manipulations. Slides used for mock injection and controls (noninjected) were prepared in the same manner, omitting the DNA drop.

### 3. Pipette Preparation.

Injection and holding pipettes were prepared from capillary tubing (Drummond Sci. Co. R6 glass 1.0 mm dia., and Leitz 1.0 mm dia., respectively) which had been previously siliconized (Sigmacote, Sigma Chemical Co.). The capillary tubes were pulled on a pipette puller (Ultrafine, Frederick Haer and Co.) to form tapered ends.

The tapered tip of the holding pipette was broken off at a diameter of 20 to 30 μ by annealing it to a heated glass bead attached to the filament of the microforge, followed by rapid cooling. The broken surface was fire-polished by holding it close to the heated filament. A bend of approximately 150° was formed by holding it close to a heated filament on a microforge (DeFonbrune MF—80). The bend facilitates control over the pipette by bringing a greater length of the tip into the focal plane of the microscope.

The injection pipette was similarly bent, but the injection tip was not broken off until later in the procedure.

### 4. Microinjection Procedure

Microinjections were performed under a Leitz Diavert microscope with Leitz micromanipulators. Holding and injection pipettes were inserted into instrument collars collected to Hamilton syringes by plastic tubing filled with paraffin oil. The two pipettes were brought into cofocus and the tip of the injection pipette was removed by touching it against the holding pipette, leaving an opening of ' 1.0 μ.

The injection pipette was lowered into the DNA drop and approximately 10 pL of the DNA solution was drawn into the pipette. The volume was estimated as described below. The injection pipette was then raised out of the DNA drop and both pipettes were lowered into the protoplast medium drop. Protoplasts were picked up individually by suction on the holding pipette and turned by nudging with the injection pipette until the nucleus was readily accessible. The injection pipette was inserted into the nucleus, and approximately 1—2 pL of DNA solution was injected.

Injection pipettes were calibrated by measuring the length and basal diameter of the portion of the pipette tip holding the injection solution using an eyepiece micrometer. The volume was assumed to be that of a cone. Estimation of the quantity injected was made by observing the meniscus between the DNA solution and oil.

Following microinjection, the injection pipette was removed, and the protoplast was placed at the bottom of the medium drop by reversing suction on the holding pipette.

Mock injections were performed as above except that no fluid was injected from the injection pipette. After all protoplasts were injected, the pipettes were raised out of the depression, and the slide was transferred to the laminar flow hood.

### 5. Hanging Drop Culture

Under a dissecting scope in the laminar flow hood, the injected protoplasts were picked up with the transfer pipette, excluding as much medium as possible. They were then deposited on the lid of a petri dish, forming a hanging drop when the lid was inverted over the original protoplast culture. The size of the hanging drop was dependent on the number of protoplasts as the flow of medium from the transfer pipette was terminated when the last protoplast was deposited. Control hanging drops were made for each experiment in the same manner using approximately the same number of noninjected protoplasts from depression slides. The hanging drop cultures were then incubated at 23±2°C in the dark.

Growth of the hanging drop cultures was monitored by observation of the drops under a dissecting microscope. As microcalli grew, medium was added to replenish nutrient supplies or changed completely to reduce the osmolarity, using a transfer pipette. For the first 1—2 weeks of culture, the microcalli were maintained in the medium in which they were precultured. Additional medium was added occasionally if the microcalli were growing rapidly. After 1—2 weeks, the medium was replaced with the same medium containing only 0.2 mg/l NAA. Every 1—2 weeks, the medium was replaced with this medium except of lower osmolarity (56 g/l sorbitol followed by 20 g/l sorbitol). When the calli reached about 1.0 mm diameter (usually after 1.5—2 months of culture), they were transferred to agar plates containing the same medium (0.6% Gibco Phytagar).

Results

Freshly isolated tobacco mesophyll protoplasts are fragile and difficult to handle with the holding pipette technique. However, it was found that partial regeneration of the cell wall by preculture of the protoplasts for a period of from 2 to 5 days, greatly improves their resilience and ability to survive microinjection. Selection of protoplasts during slide preparation results in a uniform population of cells with respect to wall regeneration and position of nucleus.

Comparison of protoplast viabilities in mock and DNA injection experiments should indicate whether injection of a DNA solution reduces protoplast viability over and above that caused by physical penetration of the injection pipette.

Viability of injected cells after three days was generally 80—90% of that of noninjected controls. Mock-injected cells averaged 82.0% (Table 1) viability whereas DNA-injected cells averaged 89.9% viability (Table 2) when compared to noninjected cells. Comparison of mean viabilities in injected and control cells (Tables 1 and 2) shows that there is a reduction in viability caused by the penetration of the cells by the injection pipette. However, the magnitude of this reduction is only 10—20%. The mean viabilities in mock injection (Table 1) and DNA injection (Table 2) experiments are very similar. This indicates that injection of picoliter quantities of an aqueous DNA solution does not further reduce viability.

Generally, 15—25 cells can be injected per hour depending on the condition of the protoplasts. This is easily doubled if injection into the nucleus is not required.

For successful culture of the protoplasts in hanging drops, maintenance of cell densities like that of normal protoplast cultures ($10^4$—$10^6$ cells per ml) is desirable. Formation of hanging drops of appropriate densities was ascertained by observation with a dissecting microscope. Culture of 10—200 cells in 0.25—20 µl hanging drops resulted in microcalli. When cultured in hanging drops, microinjected protoplasts divided to form microcalli in the same manner as the noninjected protoplasts of the controls.

## TABLE 1
### Viability of Mock-Injected Tobacco Mesophyll Protoplasts in Hanging Drops Three Days After Injection

Viability
(Viable Cells/Total Cells)

| Experiment | Mock Injected | Control | Viability as % of Control |
|---|---|---|---|
| 1 | 47% (42/90) | 74% (50/67)[++] | 64 |
| 2 | 78% (47/60) | 78% (37/48)[+] | 100 |
| Mean ± S.D. | 62.5 ± 21.9 | 75.2 ± 9.9 | 82.0 ± 25.5 |

[+] and [++] denote average viabilities of 2 and 3 replicates, respectively.

6

TABLE 2
Viability of DNA-Injected Tobacco Mesophyll Protoplasts In Hanging Drops Three Days After DNA Injection

Viability
(Viable Cells/Total Cells)

| Experiment | DNA Injected | Control | Viability as % of Control |
|---|---|---|---|
| 1 | 53 (16/30) | 73 (22/30) | 73 |
| 2 | 60 (6/10) | 75 (9/12) | 80 |
| 3 | 30 (15/50) | 33 (25/75) | 91 |
| 4 | 82 (45/55) | 50 (40/80) | 164 |
| 5 | 73 (40/55) | 83 (50/60) | 88 |
| 6 | 40 (30/75) | 65 (41/63)[+] | 62 |
| 7 | 79 (42/53) | 82 (52/64)[+] | 96 |
| 8 | 44 (35/79) | 57 (45/80)[+] | 77 |
| 9 | 66 (45/68) | 71 (50/70) | 93 |
| 10 | 54 (25/46) | 72 (43/60)[++] | 75 |
| 11 | 45 (25/55) | 45 (25/55) | 100 |
| 12 | 61 (48/79) | 85 (62/73)[+] | · 72 |
| 13 | 85 (60/71) | 79 (56/71) | 108 |
| 14 | 75 (135/180) | 80 (160/199) | 94 |
| 15 | 78 (80/120) | 85 (85/100) | 92 |
| 16 | 65 (47/72) | 83 (61/74)[++] | 78 |
| 17 | 80 (51/64) | 70 (42/60) | 114 |
| 18 | 82 (80/90) | 88 (90/102) | 93 |
| 19 | 70 (75/107) | 86 (105/123)[+] | 81 |
| 20 | 71 (50/70) | 92 (66/72)[++] | 77 |
| 21 | 53 (45/85) | 67 (55/80) | 79 |
| Mean ± S.D. | 64.1 ± 15.7 | 75.0 ± 15.7 | 89.9 ± 21.2 |

[+] and [++] denote average viabilities of 2 and 3 replicates, respectively.

The results of culturing injected cells reported here (see Tables 1 and 2) indicate that introduction of the injection pipette into a tobacco protoplast does lower viability. However, injection of picoliter quantities of an aqueous DNA solution does not further reduce viability. A reduction in viability of only 10—20% does not appear to seriously limit the usefulness of microinjection techniques for plants.

Genomic DNA integration was established in the next study. Following the procedure described above plasmids were injected into the nucleus or cytoplasm of tobacco mesophyll protoplasts. Plasmid pCGN561 (29.3 kb) was injected in about 1—2 pL at about $10^3$ copies/pL (picoliter). Plasmid pCGN561 has a HindIII fragment construct inserted into RK290. The construct has the following restriction pattern and functional sequences.

Plasmid pCGN169 (6.9 kb) was injected in about 2 pL at about $10^2$—$10^5$ copies/pL. Plasmid pCGN169 has a *Bam*HI construct fragment inserted into pUC19. The construct has the following restriction pattern and functional sequences.

The injected protoplasts were then grown to microcalli and the calli screened by the Southern technique for the present of the inserted DNA fragments.

pCGN561 Intranuclear Microinjection

| Exp. No. | No. Injected Protoplasts | No. Alive Day 3 | No. Calli Produced | No. Calli Containing 561 DNA |
|---|---|---|---|---|
| 1 | 14 | 11 | 2 | 1 |
| 2 | 21 | 19 | 1 | 0 |
| 3 | 25 | 24 | 24 | 3 |
| 4 | 20 | 15 | 15 | 2 |
| 5 | 33 | 28 | 14 | 2 |
| Total | 113 | 96 | 56 | 8 |

## pCGN561 Cytoplasmic Microinjection

| Exp. No. | No. Injected Protoplasts | No. Alive Day 3 | No. Calli Produced | No. Calli Containing 561 DNA |
|---|---|---|---|---|
| 1 | 26 | 10 | 1 | 0 |
| 2 | 31 | 22 | 13 | 1 |
| 3 | 43 | 20 | 7 | 1 |
| 4 | 33 | 21 | 5 | 1 |
| 5 | 38 | 14 | 5 | 0 |
| 6 | 32 | 18 | 3 | 0 |
| 7 | 31 | 15 | 2 | 0 |
| 8 | 40 | 28 | 13 | 0 |
| 9 | 30 | 16 | 4 | 0 |
| Total | 304 | 164 | 53 | 3 |

Summary of Southern Analysis Of Transformed
Calli from pCGN561 Microinjection

| Type of Injection | Exp. No. | Transformed Callus No. | Approximate Band Sizes (kb) | | Approximate No. Copies Per Genome |
|---|---|---|---|---|---|
| | | | 561 probe | kan probe | |
| Nuclear | 1 | 1 | 11.5 | — | ~ 1 |
| | | | 10.0 | — | |
| | | | 3.6 | 3.6 | |
| | 3 | 1 | 23.0 | — | 1.2 |
| | | | 3.5 | 3.5 | |
| | | 2 | 3.6 | 3.6 | 0.25 |
| | | 3 | 5.4 | — | 0.4 |
| | | | 1.6 | — | |
| | 4 | 1 | 5.2 | — | 0.1 |
| | | 2 | 4.9 | — | 0.25 |
| | 5 | 1 | 2.8 | — | 1.2 |
| | | 2 | 2.6 | — | 1.2 |
| Cytoplasmic | 1 | 1 | — | 4.5 | ~ 1 |
| | 2 | 1 | 3.2 | — | 0.8 |
| | | | 2.5 | — | |
| | | | 1.4 | — | |
| | 3 | 1 | 3.8 | — | ~ 1 |

According to the present invention, novel methods are provided for the microinjection of maco-molecules chromosomes, nuclei, organelles, and the like into the cytoplasm and nucleus of plant protoplasts. By culturing the protoplasts for a sufficient amount of time to partially regenerate their cell walls, the protoplasts may be manipulated without causing rupture of the fragile protoplast envelope. It has been found that microinjection may be delayed until the cell wall has substantially regenerated, but should be accomplished prior to the first cell division. Microinjection using these techniques provides greatly enhanced viability of the injected protoplasts.

Although the foregoing invention has been described in some detail by way of illustration and example for purposes of clarity of understanding, it will be appreciated that modifications and changes may be practiced within the scope of the appended claims.

**Claims**

1. A method for microinjection of macromolecules into plant protoplasts, said method comprising:
preparing plant cell protoplasts from a preselected plant material;
preculturing the protoplasts in a suitable nutrient medium for a time sufficient to partially regenerate a cell wall around the protoplast but prior to complete regeneration thereof so that the protoplast is resistant to damage during subsequent manipulations;
immobilizing the protoplasts after preculturing is complete; and
injecting the macromolecules into the protoplasts.

2. A method as in claim 1, wherein the protoplasts are precultured for at least 1 day prior to being immobilized.

3. A method as in claim 1 or 2, wherein the protoplasts are precultured for a period between 1 to 5, especially 1 to 3 days.

EP 0 175 966 B1

4. A method as in any one of the claims 1 to 3, wherein the injected protoplasts are cultured in a hanging drop culture after injection.

5. A method as in any one of the claims 1 to 4, wherein the protoplasts are immobilized by applying suction to the partially regenerated cell wall.

6. A method as in any one of the claims 1 to 5, wherein the macromolecules are polynucleotides.

7. A method for microinjection of macromolecules into plant protoplasts, said method employing a holding pipette capable of attaching to a single protoplast by suction through an orifice and an injection pipette having a tapered end capable of insertion into the protoplasts and injection of macromolecules into the interior of the protoplasts, said method comprising:

immobilizing an individual protoplast by applying a suction on the protoplast using the holding pipette, said protoplast having been precultured for a time sufficient to partially regenerate a cell wall around the protoplast but prior to complete regeneration thereof so that the protoplast is resistant to damage during subsequent manipulations;

penetrating the partially regenerated cell wall with the tapered end of the injection pipette; and

injecting macromolecules from the injection pipette into the immobilized protoplasts.

8. A method as in claim 7, wherein the protoplasts are precultured for at least 1 day prior to being immobilized.

9. A method as in claim 7 or 8, wherein the protoplasts are precultured for a period between 1 to 5, especially 1 to 3 days.

10. A method as in any one of the claims 7 to 9, wherein the protoplasts are cultured in a hanging drop culture subsequent to injection.

11. A method as in any one of the claims 7 to 10, wherein the macromolecules are polynucleotides.

12. An improved method for the microinjection of macromolecules into plant protoplasts, said method characterized by culturing the protoplasts in a suitable nutrient medium for a time sufficient to partially regenerate the cell wall but prior to first cell division, and injecting each protoplast individually while it is immobilized by applying a suction against the partially regenerated cell wall.

**Patentansprüche**

1. Verfahren zur Mikroinjektion von Makromolekülen in Pflanzenprotoplasten, umfassend die Schritte:
Herstellung von Pflanzenzellprotoplasten aus einem ausgewählten Pflanzenmaterial;
Präkultivierung der Protoplasten in einem geeigneten Nährmedium für eine zur partiellen Regeneration einer Zellwand um den Protoplasten ausreichende Zeit, jedoch vor der vollständigen Regeneration derselben, so daß der Protoplast gegen Schädigungen während anschließender Manipulationen umempfindlich ist;
Immobilisierung der Protoplasten nach Abschluß der Präkultivierung, und
Injektion der Makromoleküle in die Protoplasten.

2. Verfahren nach Anspruch 1, dadurch gekennzeichnet, daß die Protoplasten mindestens einen Tag vor der Immobilisierung präkultiviert werden.

3. Verfahren nach Anspruch 1 oder 2, dadurch gekennzeichnet, daß die Protoplasten für eine Zeit im Bereich von 1 bis 5, insbesondere von 1 bis 3 Tagen präkultiviert werden.

4. Verfahren nach einem jeden der Ansprüche 1 bis 3, dadurch gekennzeichnet, daß die injizierten Protoplasten nach der Injektion in einer hängenden Tropfenkultur kultiviert werden.

5. Verfahren nach einem jeden der Ansprüche 1 bis 4, dadurch gekennzeichnet, daß die Protoplasten durch Anlegung eines Unterdruckes an die partiell regenerierte Zellwand immobilisiert werden.

6. Verfahren nach einem jeden der Ansprüche 1 bis 5, dadurch gekennzeichnet, daß die Makromoleküle Polynucleotide sind.

7. Verfahren zur Mikroinjektion von Makromolekülen in Pflanzenprotoplasten unter Verwendung einer Haltepipette, die durch Unterdruck durch eine Öffnung an einen einzelnen Protoplasten anheftbar ist, und einer Injektionspipette mit einem zugespitzten Ende, die in die Protoplasten einführbar ist und mit der Makromoleküle in das Innere der Protoplasten injiziert werden können, umfassend die Schritte:
Immobilisierung eines einzelnen Protoplasten durch Einwirkung von Unterdruck auf den Protoplasten unter Verwendung der Haltepipette, wobei der Protoplast für eine zur partiellen Regeneration einer Zellwand um den Protoplasten ausreichenden Zeit, jedoch vor der vollständigen Regeneration desselben, präkultiviert worden ist, so daß der Protoplast gegen Schädigungen während anschließender Manipulationen umempfindlich ist;
Durchstoßen der partiell regenerierten Zellwand mit dem zugespitzten Ende der Injektionspipette, und
Injektion von Makromolekülen aus der Injektionspipette in den immobilisierten Protoplasten.

8. Verfahren nach Anspruch 7, dadurch gekennzeichnet, daß die Protoplasten mindestens einen Tag vor der Immobilisierung präkultiviert werden.

9. Verfahren nach Anspruch 7 oder 8, dadurch gekennzeichnet, daß die Protoplasten für eine Zeit im Bereich von 1 bis 5, insbesondere von 1 bis 3 Tagen präkultiviert werden.

10. Verfahren nach einem jeden der Ansprüche 7 bis 9, dadurch gekennzeichnet, daß die Protoplasten in einer hängenden Tropfenkultur nach der Injektion kultiviert werden.

11. Verfahren nach einem jeden der Ansprüche 7 bis 10, dadurch gekennzeichnet, daß die Makromoleküle Polynucleotide sind.

12. Verbessertes Verfahren für die Mikroinjektion von Makromolekülen in Pflanzenprotoplasten, gekennzeichnet durch die Kultivierung der Protoplasten in einem geeigneten Nährmedium für eine zur partiellen Regeneration der Zellwand ausreichenden Zeit, jedoch vor der ersten Zellteilung, und Injektion in jeden einzelnen Protoplasten, während dieser durch Anlegung eines Unterdruckes an die partiell regenerierte Zellwand immobilisiert ist.

**Revendications**

1. Procédé de micro-injection de macromolécules dans des protoplastes végétaux, ce procédé comprenant les opérations qui consistent:

à préparer des protoplastes de cellules végétales à partir d'un matériel végétal présélectionné;

à pré-cultiver les protoplastes dans un milieu nutritif approprié pendant un temps suffisant pour régénérer partiellement une paroi cellulaire autour du protoplaste, mais sans attendre la régénération complète de celle-ci, de telle manière que le protoplaste soit résistant à l'endommagement au cours des manipulations suivantes;

à immobiliser les protoplastes après que leur pré-culture est achevée; et

à injecter les macromolécules dans les protoplastes.

2. Procédé selon la revendication 1, dans lequel les protoplastes sont pré-cultivés pendant 1 jour au moins avant d'être immobilisés.

3. Procédé selon la revendication 1 ou 2, dans lequel les protoplastes sont pré-cultivés pendant une période comprise entre 1 et 5 jours, notamment entre 1 et 3 jours.

4. Procédé selon l'une quelconque des revendications 1 à 3, dans lequel les protoplastes injectés sont cultivés en une culture en goutte suspendue après l'injection.

5. Procédé selon l'une quelconque des revendications 1 à 4, dans lequel les protoplastes sont immobilisés par application d'aspiration à la paroi cellulaire partiellement régénérée.

6. Procédé selon l'une quelconque des revendications 1 à 5, dans lequel les macromolécules sont des polynucléotides.

7. Procédé de micro-injection de macromolécules dans des protoplastes végétaux, ce procédé utilisant une pipette de maintien capable de s'attacher à un seul protoplaste par aspiration à travers un orifice et une pipette d'injection comportant une extrémité effilée susceptible d'être insérée dans les protoplastes et d'injecter des macromolécules à l'intérieur des protoplastes, ce procédé comprenant les opérations consistant:

à immobiliser un protoplaste individuel par application d'une aspiration au protoplaste à l'aide de la pipette de maintien, ce protoplaste ayant été pré-cultivé pendant un temps suffisant pour régénérer partiellement une paroi cellulaire autour du protoplaste, mais sans attendre la régénération complète de celle-ci, de telle manière que le protoplaste soit résistant à l'endommagement pendant les manipulations suivantes;

à faire pénétrer l'extrémité effilée de la pipette d'injection à travers la paroi cellulaire partiellement régénée; et

à injecter des macromolécules dans les protoplastes immobilisés à partir de la pipette d'injection.

8. Procédé selon la revendication 7, dans lequel les protoplastes sont pré-cultivés pendant 1 jour au moins avant d'être immobilisés.

9. Procédé selon la revendication 7 ou 8, dans lequel les protoplastes sont pré-cultivés pendant une période comprise entre 1 et 5 jours, notamment entre 1 et 3 jours.

10. Procédé selon l'une quelconque des revendications 7 à 9, dans lequel les protoplastes sont cultivés en une culture en goutte suspendue après l'injection.

11. Procédé selon l'une quelconque des revendications 7 à 10, dans lequel les macromolécules sont des polynucléotides.

12. Procédé amélioré de micro-injection de macromolécules dans des protoplastes végétaux, caractérisé par les opérations consistant à cultiver les protoplastes dans un milieu nutritif approprié pendant un temps suffisant pour régénérer partiellement la paroi cellulaire, mais sans attendre la première division cellulaire, et à soumettre individuellement chaque protoplaste à l'injection, alors qu'il est immobilisé par l'application d'une aspiration contre la paroi cellulaire partiellement régénérée.